# EUROPÄISCHE PATENTSCHRIFT

(11) **EP 2 316 891 B1**
(45) Veröffentlichungstag und Bekanntmachung des Hinweises auf die Patenterteilung: **26.02.2014**
(21) Anmeldenummer: 10013293.5
(22) Anmeldetag: 04.10.2010
(51) Int. Cl.: C09C 1/00, C09C 1/28, C09C 1/30

(54) **Pigmente**
Pigments
Pigments

(30) Priorität: 29.10.2009 DE 102009051171
(43) Veröffentlichungstag der Anmeldung: 04.05.2011
(73) Patentinhaber: Merck Patent GmbH, 64293 Darmstadt (DE)
(72) Erfinder: Schmidt, Christoph Dr., 65830 Kriftel (DE); Schoen, Sabine Dr., 45701 Herten (DE)

(56) Entgegenhaltungen:
- EP-A1- 1 063 265
- EP-A1- 1 431 351
- EP-A2- 0 919 599
- US-A1- 2003 082 123

## Beschreibung

Die vorliegende Erfindung betrifft anorganische Pigmente basierend auf sphärischen Partikeln, wobei die sphärischen Partikel einen Durchmesser von < 100 µm aufweisen und auf der Oberfläche (1. Schicht) mit Agglomeraten ausgewählt aus der Gruppe ZrO₂, ZnO, Al₂O₃, TiO₂, Fe₂O₃, FeOOH und BaSO₄ oder deren Gemische belegt sind, sowie deren Verwendung in Farben, Lacken, Druckfarben, Sicherheitsdruckfarben, Kunststoffen, keramischen Materialien, Gläsern, als Tracer und insbesondere in kosmetischen Formulierungen.

Als Pigmente werden Farbmittel bezeichnet, die im Anwendungsmedium unlöslich sind. Typische Beispiele für organische Pigmente sind Phthalocyanine, Diketo-Pyrrolopyrrole, Azopigmente und Chinacridone. Von den anorganischen Pigmenten seien die verschiedenen Arten von Eisenoxiden oder auch das Ultramarin genannt. Pigmente zeichnen sich gegenüber den im Anwendungsmedium löslichen Farbstoffen im Allgemeinen durch eine höhere chemische und photochemische Beständigkeit aus.

Als eine spezielle Form von Pigmenten können Füllstoffe betrachtet werden. Bei Füllstoffen steht nicht die Funktion "Farbgebung" im Fordergrund. Bei technischen Füllstoffen sind vielmehr Faktoren wie die Erhöhung der mechanischen Stabilität, die Abriebfestigkeit, die Wetterstabilität oder auch die Herstellkosten ausschlaggebend für ihre Verwendung.

Breite Anwendung finden Füllstoffe auch in kosmetischen Formulierungen. Beispielsweise können Puder bis zu 50 % Füllstoffe basierend auf der Endformulierung enthalten. In Lippenstiften sind 10-15 % an Füllstoffen und in Emulsionen 2-6 % an Füllstoffen typische Werte. Kosmetische Füllstoffe erfüllen ganz unterschiedliche Funktionen: in Foundations vermeiden sie durch den sogenannten Mattierungseffekt einen unerwünschten Fettglanz auf der Haut, in Pudern helfen sie z.B. das Schüttverhalten oder die Auftragseigenschaften auf der Haut zu verbessern. In Deo-Produkten wird das hohe Flüssigkeitsaufnahmevermögen von einigen Füllstoffen genutzt.

Füllstoffe bzw. Pigmente in der Kosmetik müssen vor ihrer Anwendung im zu pigmentierenden System in eine Form gebracht werden, die eine leichte Dispergierung und eine reproduzierbare Farbe ermöglicht. Diese Vorbehandlungen der Pigmente, beispielsweise Mahlen, die entscheidend die Qualität des Endproduktes beeinflussen, sind zeitaufwendig und teuer. Nachteilig ist weiterhin, dass beim Benetzen die Farbe des Pigments verändert wird. Für kosmetische Formulierungen müssen die Pigmente zusätzlich ein gutes Hautgefühl besitzen, welches die klassischen Füllstoffe nur in geringem Umfang zeigen.

Füllstoffe auf der Basis sphärischer Teilchen, insbesondere SiO₂-Kugeln, werden zunehmend in der Kosmetik eingesetzt, da sie einerseits der menschlichen Haut ein natürliches Aussehen verleihen und andererseits Falten weniger sichtbar machen können.

Anorganische sphärische Füllstoffe, die mit einer farbgebenden Schicht belegt sind, sind beispielsweise aus den Offenlegungsschriften JP 62-288662, JP 11-139926, JP 11-335240 und DE 199 29 109 bekannt.

In der WO 00/15720 wird ein Pigmentgemisch basierend auf sphärischen SiO₂-Partikeln mit hoher Lichtdiffusion offenbart, wobei ein Teil der SiO₂-Kugeln mit TiO₂/SiO₂ und der andere Teil mit TiO₂ und Fe₂O₃ beschichtet ist.

Aus der WO 99/66883 werden mit Metalloxiden, wie Titan-, Eisen- oder Zinkoxid, beschichtete SiO₂-Kugeln beschrieben, die eine finale SiO₂-Schicht aufweisen. Die so beschichteten SiO₂-Kugeln werden als Gemisch mit Interferenzpigmenten in kosmetischen Formulierungen eingesetzt.

Die aus dem Stand der Technik bekannten Füllstoffe basierend auf SiO₂-Kugeln, z. B. bekannt aus der EP 0 919 599 A2, US 2003/082123 A1, EP 1 063 265 A1 und EP 1 431 351 A1, zeigen ein relativ gutes Hautgefühl, besitzen aber den Nachteil, dass sie ein zu hohes Streuvermögen aufweisen. Der Grund dafür ist in der Struktur der Metalloxidschichten der kugelförmigen Füllstoffe zu suchen. Die funktionellen Pigmente aus dem Stand der Technik bestehen in der Regel aus sehr kleinen Partikeln, d. h., sie weisen Partikelgrößen von 0,5 - 100 nm auf, die in gleichmäßiger Anordnung die Oberfläche der Trägerkugeln belegen. An der Schichtoberfläche wird das Licht reflektiert, wodurch Glanz hervorgerufen wird. Gleichzeitig findet aber auch ein erhebliches Maß von Streuung statt, da sich auf der Schicht Einzelpartikel bilden, die als starke Streuzentren wirken. Als Folge dieser zwei gegenläufigen Effekte (Glanz und Streuung) wirken die Pigmente weiß und unnatürlich auf der Haut.

Aufgabe der vorliegenden Erfindung ist es daher ein funktionelles Pigment bereit zu stellen, das neben einem guten Hautgefühl, gleichzeitig eine gute Dispergierbarkeit in kosmetischen Formulierungen, chemische und photochemische Stabilität und eine reine Farbe besitzt. Darüber hinaus soll das Pigment im Hautauftrag als reines Pulver, in Cremes, Emulsionen, Foundations u.ä., ein weiches und ebenmäßiges und natürliches Erscheinungsbild der Haut zeigen. Es ist weiterhin erwünscht, dass das Pigment insbesondere flüssigen und pastösen Zubereitungen eine leichte Erhöhung der Festigkeit verleiht. Dadurch wird das Applikationsverhalten der Zubereitung wesentlich verbessert. Die Viskosität sollte durch das Pigment in der für die jeweilige Anwendung gewünschten Weise optimiert werden. Durch eine leicht thixotrope Funktion des Pigments wird die Verteilbarkeit der kosmetischen Zubereitungen auf der Haut deutlich erleichtert. So können hochviskose Cremes, also feste Foundations, hergestellt werden, die dennoch eine sehr gute Verteilbarkeit auf der Haut bzw. ein sehr gutes Abtragsverhalten bei der Entnahme aus dem Container besitzen.

Neben diesen Produkteigenschaften ist eine einfache technische Herstellung des Pigments eine weitere Aufgabe der Erfindung.

Auch die Einstellung bestimmter Eigenschaften, wie z.B. das Deckvermögen und die Farbintensität, des Pigments sollen im Rahmen des Herstellungsverfahrens in einfacher Weise kontrolliert werden können.

Überraschenderweise wurde gefunden, dass Pigmente basierend auf sphärischen Partikeln mit einen Durchmesser von 0,1 - 100 µm, die auf der Oberfläche (1. Schicht) mit Agglomeraten ausgewählt ausgewählt aus der Gruppe ZrO₂, ZnO, Al₂O₃, TiO₂, Fe₂O₃, Fe₃O₄, FeOOH und BaSO₄ oder deren Gemische beschichtet sind, eine bessere Einstellung des Deckvermögens und ein deutlich natürlicheres Erscheinungsbild der Haut erlauben und zudem ein besseres Hautgefühl als die Füllstoffe/Pigmente aus dem Stand der Technik aufweisen. Darüber hinaus zeigen die erfindungsgemäßen Pigmente den gewünschten Einfluss auf die Textur, d.h. sie wirken leicht erhöhend auf die Viskosität von Emulsionen und die Festigkeit von Foundations, ohne die Applikationseigenschaften ungünstig zu beeinflussen.

Gegenstand der Erfindung sind daher Pigmente, die sich dadurch auszeichnen, dass sie auf sphärischen Partikeln basierend, und die sphärischen Partikel ausgewählt sind aus der Gruppe Magnesiumsilikat, Aluminiumsilikat, Alkalialuminiumsilikate, Erdalkalialuminiumsilikate und deren Kombinationen und einen Partikeldurchmesser von 0,1 - 100 µm aufweisen und auf der Oberfläche (1. Schicht) mit Agglomeraten ausgewählt aus der Gruppe ZrO₂, ZnO, Al₂O₃, TiO₂, Fe₂O₃, Fe₃O₄, FeOOH oder mit BaSO₄ oder Gemische davon beschichtet sind, und optional mit einer weiteren Schicht (2. Schicht) aus einem Metalloxid oder Metalloxidgemisch, einem Farblack oder Berliner Blau belegt sind.

Die erfindungsgemäßen sphärischen Pigmente zeigen gegenüber den bekannten Pigmenten
- ein natürlicheres Erscheinungsbild der Haut
- eine leichtere Dispergierbarkeit
- eine verbesserte Verarbeitbarkeit
- ein in weiten Grenzen einstellbares Deckvermögen
- ein verbessertes Hautgefühl
- eine verbesserte Textur der kosmetischen Zubereitungen
- ein verbessertes Applikationsverhalten der kosmetischen Formulierungen
- einen höheren Weißgrad
- eine höhere Farbreinheit.

Gegenstand der Erfindung ist weiterhin die Verwendung der erfindungsgemäßen sphärischen Pigmente in Farben, Lacken, vorzugsweise in Industrielacken, Druckfarben, Sicherheitsdruckfarben, Kunststoffen, keramischen Materialien, Gläsern, als Tracer, als Füllstoff und insbesondere in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Chips, Pellets, Würstchen, Briketts, etc., geeignet. Die Trockenpräparate finden insbesondere Anwendung in Druckfarben und in der Kosmetik.

Geeignete Basissubstrate sind sphärische Partikel, wie sie z. B. im Handel angeboten werden, u.a. von Sunjin Chemical, Omega Materials, 3M, Dow Corining oder Evonik. Die sphärischen Partikel sind ausgewählt aus der Gruppe Magnesiumsilikat, Aluminiumsilikat, Alkalialuminiumsilikate, Erdalkalialuminiumsilikate und deren Kombinationen.

Besonders bevorzugte sphärische Substrate sind Magnesium- oder Alkalialuminiumsilikate, wie sie beispielsweise von der Fa. 3M unter den Markennamen Ceramic Microspheres und Cosmetic Microspheres bzw. Zeeospheres vertrieben werden.

Als Substrate können auch Gemische von unterschiedlichen Substraten zum Einsatz kommen. Vorzugsweise werden nicht mehr als zwei unterschiedliche Substrate verwendet. Besonders bevorzugte Substratgemische sind:
- Alkalialuminiumsilikat und Magnesiumsilikat
- Alkalialuminiumsilikat und Siliziumdioxid
- Alkalialuminiumsilikat und Erdalkalialuminiumsilikat.

Die mikroskaligen sphärischen Basissubstrate der erfindungsgemäßen Pigmente besitzen einen Teilchendurchmesser von 0,1 - 100 µm, vorzugsweise von 0,3 - 50 µm, insbesondere von 0,5 - 15 µm.

Die mikroskaligen sphärischen Basispartikel sind auf der Oberfläche (1. Schicht) mit Agglomeraten beschichtet, wobei es sich vorzugsweise um mindestens ein Metalloxid handelt, insbesondere aus der Gruppe ZrO₂, ZnO, Al₂O₃, TiO₂, Fe₂O₃, Fe₃O₄, FeOOH oder deren Gemische. Weiterhin kann das Agglomerat auch aus BaSO₄ bestehen bzw. einem Gemisch aus BaSO₄ und einem oder mehreren der genannten Metalloxide.

Vorzugsweise weisen die Basispartikel eine Beschichtung von Agglomeraten aus TiO₂ auf. Das TiO₂ kann dabei in der Rutil- oder in der Anatas-Modifikation vorliegen, vorzugsweise liegt es als Anatas-Form vor.

Unter Beschichtung(en) sind in dieser Patentanmeldung die teilweise oder vollständige Belegung der Basispartikel mit Metalloxiden und/oder mit BaSO₄, wobei sowohl das bzw. die Metalloxide oder das BaSO₄ in Form von Agglomeraten vorliegen, zu verstehen.

Unter Agglomeraten sind in dieser Anmeldung unregelmäßig auf der Oberfläche der sphärischen Basispartikel verteilte Oxid- bzw. BaSO₄-Teilchen zu verstehen. Durch die Bildung von Agglomeraten wird die Zahl der Lichtstreuzentren verringert, und das Streuvermögen der Beschichtung steigt mit zunehmendem Belegungsgrad weniger stark an bei einer Belegung mit nicht aggregierten Einzelpartikeln. Die teilweise vertikale Anordnung mehrerer Oxidteilchen zu Agglomeraten führt auch zu einer unregelmäßigen Schichtdicke der Beschichtung und einer zerklüfteten Oberfläche. Die Wechselwirkung mit dem Medium ist daher intensiver und es können sich zwischen den beschichten sphärischen Partikeln Netzwerke bilden, die eine gewünschte Viskositätserhöhung und Verbesserung der Festigkeit einer Foundation bewirken und gleichzeitig das Applikationsverhalten verbessern.

Die mittlere Schichtdicke der ersten Schicht aus den sphärischen Partikeln beträgt vorzugsweise 0,01 - 2 µm, insbesondere von 0,02 - 1 µm, ganz besonders bevorzugt von 0,05 - 0,8 µm.

Da die Agglomerate sehr unregelmäßig auf der Oberfläche verteilt sind, wird in dieser Anmeldung die mittlere Schichtdicke der Agglomerate angegeben, d.h. eine Schichtdicke bei einer angenommenen gleichmäßigen und kompakten (nicht agglomerierten) Verteilung des Schichtmaterials auf der Substratoberfläche.

Häufig empfiehlt es sich zur Verbesserung der Dispergierbarkeit, der chemischen und photochemischen Stabilität und des Hautgefühles eine Deckschicht (finale Schicht) aus SiO₂ zu applizieren. Die SiO₂-Schicht besitzt in der Regel eine mittlere Schichtdicke von 0,01 - 1 µm, insbesondere von 0,02 - 0,7 µm und ganz besonders bevorzugt von 0,05 - 0,5 µm.

Diese finale SiO₂-Schicht umhüllt die mit Agglomeraten belegten sphärischen Partikel in dünner Schicht. Sie verbessert die Dispergierbarkeit und verhindert chemische und photochemische Wechselwirkungen des erfindungsgemäßen Pigments mit dem Anwendungsmedium.

Die sphärischen Basispartikel werden vorzugsweise mit einer oder zwei Schichten belegt. Insbesondere bevorzugt ist die Belegung der sphärischen Basispartikel mit einer farblosen ersten Schicht, wie z.B. TiO₂ oder zur Einstellung der Körperfarbe mit einem Gemisch aus TiO₂/Fe₂O₃ und optional mit einer Deckschicht aus SiO₂.

Optional kann auf die erste Beschichtung auch eine zweite Beschichtung aus einem Metalloxid, vorzugsweise Fe₂O₃ oder Fe₃O₄, einem Farblack oder Berliner Blau aufgebracht werden. Bei der zweiten Beschichtung kann es sich auch um ein Metalloxidgemisch handeln, bestehend aus zwei, drei oder mehr Metalloxiden. Vorzugsweise besteht das Metalloxidgemisch aus zwei Metalloxiden, insbesondere aus TiO₂ und Fe₂O₃.

Farblacke sind organische Farbmittel, die an eine anorganische Trägermatrix gebunden sind. Als Trägermatrix kommen insbesondere netzwerkbildende anorganische Oxide in Frage, wie z.B. Al₂O₃ und SiO₂. Als organische Farbmittel sind alle verlackbaren farbigen Verbindungen zu nennen, insbesondere die Alkali- und Erdalkalisalze von Karminrot (CAS-Nr. 1390-65-4), Allura Rot (CAS Nr. 25956-17-6), Tartrazin (CAS-Nr. 1934-21-0), Brillantblau FCF (CAS-Nr. 3844-45-9), Erythrosin (CAS-Nr. 16423-68-0) und Phloxin B (CAS-Nr. 18472-87-2). Diese Auflistung gibt nur eine kleine Zahl der möglichen Farblacke wieder und ist nicht einschränkend zu verstehen.

Berliner Blau ist das Eisen(III)-salz des Hexacyanoferrat(II)-Anions mit der Summenformel Fe₄[Fe(CN)₆]₃. Es lässt sich leicht durch Vereinigung der Lösungen von Gelbem Blutlaugensalz und Eisen(III)-Salzen oder Rotem Blutlaugensalz und Eisen(II)-Salzen in schwach saurer Lösung herstellen.

Die mittleren Schichtdicken der zweiten Schicht aus Metalloxid, Farblack bzw. Berliner Blau liegen bei 10 - 500 nm, bevorzugt bei 10 - 200 nm und besonders bevorzugt bei 10 - 100 nm. Der Gewichtsanteil des Farblackes am Gesamtpigment ist von der gewünschten Farbintensität des Produktes und dem Färbungseffekt des jeweiligen Farblackes abhängig. Er beträgt 0,1 - 30 %, bevorzugt 0,2 - 25 % und besonders bevorzugt 0,5 - 20 %.

Besonders bevorzugte erfindungsgemäße Pigmente weisen folgende Belegung auf der Oberfläche auf:
sphärisches Basissubstrat + TiO₂
sphärisches Basissubstrat + TiO₂ + SiO₂
sphärisches Basissubstrat + TiO₂/Fe₂O₃
sphärisches Basissubstrat + TiO₂/Fe₂O₃ + SiO₂
sphärisches Basissubstrat + Fe₂O₃ + SiO₂
sphärisches Basissubstrat + FeO(OH) + SiO₂
sphärisches Basissubstrat + Fe₃O₄ + SiO₂
sphärisches Basissubstrat + TiFe₂O₅ + SiO₂
sphärisches Basissubstrat + TiO₂ + Fe₂O₃ + SiO₂
sphärisches Basissubstrat + TiO₂ + Fe₃O₄ + SiO₂
sphärisches Basissubstrat + TiO₂ + Fe₄[Fe(CN)₆]₃
sphärisches Basissubstrat + TiO₂ + Fe₄[Fe(CN)₆]₃ + SiO₂
sphärisches Basissubstrat + TiO₂ + Carmin-Lack
sphärisches Basissubstrat + TiO₂ + Carmin-Lack + SiO₂.

Besonders bevorzugt sind Pigmente, die auf Al- oder Mg-Silikat-Kugeln basieren und eine Belegung mit einem Metalloxid aufweisen, vorzugsweise TiO₂ oder Fe₂O₃ und optional eine Deckschicht (finale Schicht) aus SiO₂.

Durch die finale SiO₂-Schicht bei den erfindungsgemäßen Pigmenten können die optischen Eigenschaften der Pigmente in Farbe, Farbreinheit und Farbstärke sowie die anwendungstechnischen Eigenschaften, wie z.B. Dispergierbarkeit, Hautgefühl und chemische und photochemische Stabilität, verbessert werden.

Die erfindungsgemäßen sphärischen Pigmente weisen in der Regel eine Ölzahl von 10 - 200, insbesondere von 20 - 200, ganz besonders bevorzugt von 50 - 150, auf. Die Ölzahl in dieser Patentanmeldung wird bestimmt nach DIN ISO 787/5-1980 (E).

Die per Stickstoffabsorption bestimmte BET-Oberfläche der erfindungsgemäßen Pigmente beträgt in der Regel 1 - 200, vorzugsweise 2 - 150, insbesondere 3 - 100 m²/g. Die BET-Oberfläche in dieser Patentanmeldung wird bestimmt nach DIN ISO 9277: 2003-05.

Die erfindungsgemäßen Pigmente verbessern insbesondere die Textur von Kosmetika in der Weise, dass sie ein erleichtertes Auftragen und eine gleichmäßigere Verteilung auf der Haut erzielen und das Hautgefühl verbessern. Da die Pigmente auf mineralischer Basis aufgebaut sind und überwiegend anorganische Komponenten enthalten, sind sie sehr gut auf der Haut verträglich.

Die Herstellung von sphärischen Partikeln ist bekannt. So werden beispielsweise Mg- oder Al-Silikat-Kugeln in der Regel durch Erhitzen der feinteiligen silikatischen Rohstoffe und/oder ihrer oxidischen Ausgangsverbindungen in einem Gastrom zum Schmelzen gebracht und nehmen dadurch Kugelform an. Zur Erleichterung des Aufschmelzens durch Schmelzpunkterniedrigung werden dem Reaktionsgemisch typischerweise feinteilige Alkali- und/oder Erdalkali-Verbindungen zugesetzt.

Die Belegung der sphärischen Basispartikel kann in einem Eintopfverfahren erfolgen. Die erfindungsgemäßen Pigmente lassen sich auf verschiedene Art und Weisen relativ einfach herstellen. Die sphärischen Partikel können durch nasschemische Beschichtung oder im CVD- oder PVD-Verfahren mit einer oder mehreren Beschichtungen belegt werden. Vorzugsweise erfolgt die Belegung der sphärischen Basispartikel im naßchemischen Verfahren durch hydrolytische Abscheidung der Metalloxide bzw. Metallhydroxide aus ihren Salzlösungen. Die Bildung von Agglomeraten kann durch geeignete Wahl der Fällungsbedingungen hervorgerufen werden. Dies sind insbesondere die Reaktionstemperatur, der pH-Wert, die Rührgeschwindigkeit und die Dosiergeschwindigkeit der Salzlösungen. TiO₂-Fällungen werden typischerweise im pH-Bereich von 1,0 und 3,0 durchgeführt. Von pH 1,0 bis pH 3,0 nimmt die Neigung zur Agglomeratbildung zu. Wenn der pH jedoch zu hoch gewählt wird kann es zu einer sog. Nebenfällung kommen, d.h. die TiO₂-Partikel fallen "neben" die Basispartikel und bilden keine Schicht. Für die genannten Basispartikel sind aufgrund des unterschiedlichen Oberflächenverhaltens in der Regel unterschiedliche Fällungsbedingungen zu wählen, die für den Fachmann auf dem Gebiet der Pigmente leicht zu ermitteln sind.

Gegenstand der Erfindung ist auch ein Verfahren zur Herstellung der erfindungsgemäßen Pigmente, wobei die sphärischen Partikel durch Ausfällung des Schichtmaterials in wässriger Suspension durch weitgehend gleichmäßige und gleichzeitige oder zeitversetzte Zugabe der Lösungen der Agglomerat- bzw. der Schicht-bildenden Rohstoffe in Gegenwart von Säuren bzw. Basen mit den Agglomeraten belegt, abfiltriert, gewaschen, getrocknet, gegebenenfalls kalziniert und gesiebt werden.

Die Metalloxidschichten auf den sphärischen Partikeln werden vorzugsweise nasschemisch aufgebracht, wobei in der Regel die zur Herstellung von Perlglanzpigmenten entwickelten nasschemischen Beschichtungsverfahren angewendet werden können. Derartige Verfahren sind z. B. beschrieben in U.S. 3087828, U.S. 3087829, U.S. 3553001, DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, DE 196 18 568, EP 0 659 843, oder auch in weiteren dem Fachmann bekannten Patentdokumenten und sonstigen Publikationen.

Bei der nasschemischen Beschichtung werden die sphärischen Partikel in Wasser suspendiert und mit einem oder mehreren hydrolysierbaren Metallsalzen in einem für die Hydrolyse geeigneten pH-Wert versetzt, der so gewählt wird, dass die Metalloxide bzw. Metalloxidhydrate direkt auf den Kugeln als Agglomerate ausgefällt werden. Der pH-Wert wird üblicherweise durch gleichzeitiges Zudosieren einer Base oder Säure konstant gehalten. Anschließend werden die Pigmente abgetrennt, gewaschen und getrocknet, vorzugsweise bei 50-350 °C, insbesondere bei 80-150 °C, und gegebenenfalls kalciniert, wobei die Glühtemperatur in Hinblick auf die jeweils vorliegende Beschichtung optimiert werden kann. In der Regel liegen die Glühtemperaturen zwischen 250 und 1100 °C, vorzugsweise zwischen 350 und 900 °C. Falls gewünscht können die Pigmente nach Aufbringen einzelner Beschichtungen abgetrennt, gewaschen und ggf. geglüht werden um dann zur Auffällung der weiteren Schichten wieder resuspendiert zu werden.

Weiterhin kann die Beschichtung auch in einem Wirbelbettreaktor durch Gasphasenbeschichtung erfolgen, wobei z. B. die in EP 0 045 851 und EP 0 106 235 zur Herstellung von Perlglanzpigmenten vorgeschlagenen Verfahren entsprechend angewendet werden können. Im Fall, dass die farbgebenden Schichten Fe₃O₄ oder andere reduzierte Oxidtypen enthalten, kann ein Reduktionsschritt, z. B. Glühung in reduzierender Atmosphäre, als letzter Verfahrenschritt erforderlich sein.

Für das Aufbringen einer finalen SiO₂-Schicht wird bevorzugt das in der DE 196 18 569 beschriebene Verfahren verwendet. Zur Herstellung der SiO₂-Schicht wird vorzugsweise Natrium- oder Kaliumwasserglaslösung eingesetzt.

Bei einer Bariumsulfat-Fällung werden Lösungen eines Bariumsalzes, z.B. Bariumchlorid, und eines Sulfates, z.B. Natriumsulfat, gleichzeitig zudosiert. Bei beiden Verfahren bilden sich Oxid- bzw. Sulfatkeime in der Lösung und schlagen sich auf der Oberfläche der sphärischen Partikel als Agglomerate nieder.

Wenn die gewünschte Menge des Schichtmaterials aufgefällt ist, wird die Reaktion unterbrochen und ein für die Aufarbeitung geeigneter pH-Wert, z.B. pH 5, eingestellt.

Zur Aufarbeitung werden die beschichteten sphärischen Partikel abfiltriert, mit Wasser gewaschen und vorzugsweise bei Temperaturen von 50 - 350 °C für eine Dauer von 1 - 20 h getrocknet und gegebenenfalls bei Temperaturen von 350 - 1100 °C für 0,1 - 2 h kalziniert. Abschließend wird das Pigment gesiebt.

Der Farbton und das Deckvermögen der erfindungsgemäßen Pigmente kann in weiten Grenzen durch unterschiedliche Wahl der Beschichtungsstoffe und der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Im Fall der Beschichtung mit Farblacken wird in der Regel zunächst eine Matrix von Siliziumdioxid, Aluminiumoxid oder einer Mischung von beiden aufgebracht. Dies geschieht beispielsweise nach den in DE 2429762 und EP 1595921 beschriebenen Verfahren durch Hydrolyse von Silicat- bzw. Aluminat-Lösungen. In einem zweiten Schritt wird die organische Komponente des Farblackes in gelöster Form in Gegenwart von geeigneten Kationen von z.B. Calcium, Aluminium oder Barium auf die Matrix aufgezogen, wie z.B. in der EP 1595921 beschrieben.

Im Falle von Berliner Blau wird die wässrige Pigmentsuspension mit Lösungen von Gelbem Blutlaugensalz und einer Eisen(II)sulfat-Lösung in Gegenwart eines Oxidationsmittels versetzt. Bei Verwendung von Eisen(III)-Lösung, z.B. Eisen(III)chlorid, kann auf die Zuführung eines Oxidationsmittels verzichtet werden. Alternativ zu Gelbem Blutlaugensalz kann auch Rotes Blutlaugensalz eingesetzt werden, wobei eine Eisen(II)-verbindung, z.B. Eisen(II)sulfat-Lösung, zugesetzt werden muss. Entsprechende Verfahren zur Durchführung von Berliner Blau-Fällungen sind z.B. in DE 2313332, EP 1595921, EP 1254928 und EP 0659843 beschrieben.

Zur Erhöhung der Licht-, Wasser- und Wetterstabilität empfiehlt es sich häufig, in Abhängigkeit vom Einsatzgebiet, das fertige Pigment bzw. den fertigen Füllstoff einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung auf die finale SiO₂-Schicht wird die chemische und photochemische Stabilität weiter erhöht oder die Handhabung des Absorptionspigments, insbesondere die Einarbeitung in unterschiedliche Medien erleichtert. Zur Verbesserung der Benetzbarkeit, Dispergierbarkeit und/oder Verträglichkeit mit den Anwendermedien können funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493.

Die erfindungsgemäßen Pigmente sind mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen, sowie für Anwendungen im Agrarbereich, z. B. für Gewächshausfolien, sowie z. B. für die Farbgebung von Zeltplanen, geeignet.

Die vorliegende Erfindung erlaubt die Herstellung besonders farbreiner Pigmente mit außergewöhnlich hohem Deckvermögen. Mit den erfindungsgemäßen Pigmenten lassen sich daher besonders wirksame Effekte in den verschiedenen Anwendungsmedien erzielen, z. B. in kosmetischen Formulierungen, wie z. B. Nagellacken, Lippenstiften, Presspudern, Gelen, Lotionen, Seifen, Zahnpasta, in Lacken, in Industrielacken und Pulverlacken, sowie in Kunststoffen und in der Keramik.

Aufgrund des guten Hautgefühls und der sehr guten Hautadhäsion sind die erfindungsgemäßen Pigmente insbesondere als Füllstoff in der dekorativen Kosmetik, aber auch für Personal Care Applications, wie z.B. Body Lotions, Emulsionen, Seifen, Shampoos, etc., geeignet.

Die erfindungsgemäßen Pigmente weisen eine hohe spezifische Oberfläche auf. Dadurch sind sie in besonderer Weise für die Verwendung in Kosmetika geeignet, bei denen flüssigkeitsabsorbierende Eigenschaften gewünscht werden, wie z.B. in Deodorantien und mattierenden Cremes.

Es versteht sich von selbst, dass für die verschiedenen Anwendungszwecke die erfindungsgemäßen Pigmente auch vorteilhaft in Abmischung mit z. B.
- Metalleffektpigmenten, z. B. auf der Basis von Eisen- oder Aluminiumplättchen;
- Perlglanzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- Interferenzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- goniochromatischen Pigmenten;
- Mehrschichtpigmenten (enthaltend vorzugsweise 2, 3, 4, 5 oder 7 Schichten) auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- organischen Farbstoffen;
- organischen Pigmenten;
- anorganischen Pigmenten, wie z.B. transparenten und deckenden Weiß-, Bunt- und Schwarzpigmenten;
- plättchenförmigen Eisenoxiden;
- holographischen Pigmenten;
- LCPs (Liquid Crystal Polymers);
- Ruß
eingesetzt werden können.

Die erfindungsgemäßen Pigmente können in jedem Verhältnis mit handelsüblichen Pigmenten und/oder weiteren handelsüblichen Füllstoffen gemischt werden.

Als handelsübliche Füllstoffe sind z.B. zu nennen natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, Bornitrid sowie physikalische oder chemische Kombinationen dieser Stoffe. Bezüglich der Partikelform des Füllstoffes gibt es keine Einschränkungen. Sie kann den Anforderungen gemäß z.B. plättchenförmig, sphärisch oder nadelförmig sein.

Selbstverständlich können die erfindungsgemäßen Pigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe, wie z.B. Bentonite, Hektorite, Siliciumdioxide, Ca-Silicate, Gelatinen, hochmolekulare Kohlenhydrate und/oder oberflächenaktive Hilfsmittel, etc.

Die die erfindungsgemäßen Pigmente enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Pigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Pigmente in der Formulierung sind keine Grenzen gesetzt. Sie können - je nach Anwendungsfall - zwischen 0,001 (rinse-off-Produkte, z.B. Duschgele) - und 60 % liegen. Die erfindungsgemäßen Pigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E, etc.), Selbstbräuner (z.B. DHA, Erytrolose, u.a.) sowie weitere kosmetische Wirkstoffe, wie z.B. Bisabolol, LPO, Ectoin, Emblica, Allantoin, Bioflavanoide und deren Derivate.

Gegenstand der vorliegenden Erfindungen sind ebenfalls Formulierungen, insbesondere kosmetische Formulierungen, die neben dem erfindungsgemäßen Pigment mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsions-stabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Gegenstand der Erfindung ist auch die Verwendung der erfindungsgemäßen Pigmente in Farben, Lacken, Druckfarben, Sicherheitsdruckfarben, Kunststoffen, keramischen Materialien, Gläsern, als Tracer, in kosmetischen Formulierungen und zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1: Mit Titandioxid und Siliziumdioxid beschichtete keramische Mikrokugeln

250 g sphärische Silica-Alumina-Keramik-Kugeln (Zeeospheres W-210, 1-12 µm; Hersteller 3M) werden in 1,8 l vollentsalztem Wasser suspendiert und unter starkem Rühren auf 75 °C erhitzt. Zu dieser Mischung wird bei pH 2,2 eine Menge von 395 g TiCl₄-Lösung (30 %ig) mit einer Geschwindigkeit von 3,3 ml/min zudosiert und der pH-Wert mit Natronlauge (32 %) konstant gehalten. Danach wird der pH-Wert mit Natronlauge (32 %) auf 8,0 eingestellt und bei diesem pH-Wert 383 g Natronwasserglaslösung (13 % SiO₂, Fa. Merck KGaA) mit einer Geschwindigkeit von 1,7 ml/min zudosiert. Der pH-Wert wird mit Salzsäure (18 %) konstant gehalten.

Zur Aufarbeitung wird das Pigment abfiltriert, mit 15 l vollentsalztem Wasser gewaschen, bei 110°C 16 Stunden getrocknet und durch ein Sieb mit 32 µm Maschenweite gesiebt.

Man erhält ein rein weißes Pulver mit sehr weichem Hautgefühl, das hervorragend als Füllstoff für kosmetische Formulierungen, z.B. Presspuder, Lippenstifte und rein weiße Emulsionen und Cremes, geeignet ist.

### Beispiel 2: Mit Titandioxid und Siliziumdioxid beschichtete Magnesiumsilikat-Kugeln

250 g sphärisches Magnesiumsilikat (Cosmetic Microspheres CM-111; 1-12 µm; Hersteller 3M) werden in 1,8 l vollentsalztem Wasser suspendiert und unter starkem Rühren auf 75 °C erhitzt. Zu dieser Mischung wird bei pH 2,2 eine Menge von 395 g TiCl₄-Lösung (30 %ig) mit einer Geschwindigkeit von 3,3 ml/min zudosiert und der pH-Wert mit Natronlauge (32 %) konstant gehalten. Danach wird der pH-Wert mit Natronlauge (32 %) auf 8,0 eingestellt und bei diesem pH-Wert 383 g Natronwasserglaslösung (13 % SiO₂, Fa. Merck KGaA) mit einer Geschwindigkeit von 1,7 ml/min zudosiert. Der pH-Wert wird mit Salzsäure (18 %) konstant gehalten.

Zur Aufarbeitung wird das Pigment abfiltriert, mit 15 l vollentsalztem Wasser gewaschen, bei 110 °C 16 Stunden getrocknet und durch ein Sieb mit 32 µm Maschenweite gesiebt.

Man erhält ein weißes Pulver mit weichem Hautgefühl, das hervorragend als Füllstoff für kosmetische Formulierungen, z.B. Presspuder und Lippenstifte, geeignet ist.

### Beispiel 3: Mit Bariumsulfat und Siliziumdioxid beschichtete Magnesiumsilikat-Kugeln

200 g sphärisches Magnesiumsilikat (Cosmetic Microspheres CM-111; 1-12 µm; Hersteller 3M) werden in 1,8 l vollentsalztem Wasser suspendiert und unter starkem Rühren auf 75°C erhitzt. Zu dieser Mischung werden 20,9 g BaCl₂ x 2 H₂O gegeben und 15 Minuten nachgerührt. Anschließend werden 250 g einer Natriumsulfatlösung (w(Na₂SO₄) = 10 %) über einen Zeitraum von ca. 80 Min. zudosiert. Danach wird der pH-Wert mit Natronlauge (32 %) auf 8,0 eingestellt und bei diesem pH-Wert eine Lösung von 118 ml Natronwasserglaslösung (ca. 27 w-% SiO₂, Merck KGaA) in 159 ml vollentsalztem Wasser mit einer Geschwindigkeit von 1,7 ml/min zudosiert. Der pH-Wert wird mit Salzsäure (18 %) konstant gehalten.

Zur Aufarbeitung wird das Pigment abfiltriert, mit 15 l vollentsalztem Wasser gewaschen, bei 110 °C 16 Stunden getrocknet und durch ein Sieb mit 32 µm Maschenweite gesiebt.

Man erhält ein weißes Pulver mit gutem Hautgefühl, das hervorragend als Füllstoff für kosmetische Formulierungen, z.B. Presspuder und Lippenstifte, geeignet ist.

### Beispiel 4: Mit Bariumsulfat und Siliziumdioxid beschichtete keramische Mikrokugeln

200 g Mikrokugeln (Zeeospheres W-210, 1-12 µm, Hersteller 3M) werden in 1,8 l vollentsalztem Wasser suspendiert und unter starkem Rühren auf 75 °C erhitzt. Zu dieser Mischung werden 10,5 g BaCl₂ x 2 H₂O gegeben und 15 Minuten nachgerührt. Anschließend werden 125 g einer Natriumsulfatlösung (w(Na₂SO₄) = 10 %) über einen Zeitraum von ca. 40 Min. zudosiert. Danach wird der pH-Wert mit Natronlauge (32 %) auf 8,0 eingestellt und bei diesem pH-Wert eine Lösung von 118 ml Natronwasserglaslösung (ca. 27 w-% SiO₂, Fa. Merck KGaA) in 159 ml vollentsalztem Wasser mit einer Geschwindigkeit von 1,7 ml/min zudosiert. Der pH-Wert wird mit Salzsäure (18 %) konstant gehalten.

Zur Aufarbeitung wird das Pigment abfiltriert, mit 15 l vollentsalztem Wasser gewaschen, bei 110°C 16 Stunden getrocknet und durch ein Sieb mit 32 µm Maschenweite gesiebt.

Man erhält ein rein weißes Pulver mit sehr weichem Hautgefühl, das hervorragend als Füllstoff für kosmetische Formulierungen, z.B. Presspuder, Lippenstifte und rein weiße Emulsionen und Cremes, geeignet ist.

### Beispiel 5: Mit Titandioxid und Karminfarblack und Siliziumdioxid beschichtete Mikrokugeln

250 g sphärische Silica-Alumina-Keramik-Kugeln (Zeeospheres W-210, 1-12 µm; Hersteller 3M) werden in 1,8 l vollentsalztem Wasser suspendiert und unter starkem Rühren auf 75 °C erhitzt. Zu dieser Mischung wird bei pH 2,2 eine Menge von 395 g TiCl₄-Lösung (30 %ig) mit einer Geschwindigkeit von 3,3 ml/min zudosiert und der pH-Wert mit Natronlauge (32 %) konstant gehalten.

Nach einer Nachrührzeit von 15 Min. wird der pH-Wert mit Natronlauge (32 %) auf 7,5 eingestellt und 100 g Natronwasserglas-Lösung (5 % SiO₂) mit einer Geschwindigkeit von 3,3 ml/min. zudosiert. Der pH-Wert wird mit Salzsäure (10 % HCl) konstant gehalten und nach beendeter Zugabe 60 min. nachgerührt. Anschließend wird der pH-Wert mit Salzsäure (10 % HCl) auf 5,4 abgesenkt. Bei diesem pH-Wert werden gleichzeitig eine Lösung von 4,0 g Carmin in 350 ml Natronlauge (1,1 % NaOH) und eine Lösung von 14,12 g CaCl₂ x 2 H₂O und 3,53 g AlCl₃ x 6 H₂O in 350 ml vollentsalztem Wasser mit einer Dosierrate von jeweils 5,5 ml/min. zudosiert. Der pH-Wert wird dabei mit Salzsäure (10% HCl) konstant bei 5,4 gehalten. Danach wird der pH-Wert mit Natronlauge (32 %) auf 8,0 eingestellt und bei diesem pH-Wert 383 g Natronwasserglaslösung (13 % SiO₂, Merck KGaA) mit einer Geschwindigkeit von 1,7 ml/min zudosiert. Der pH-Wert wird mit Salzsäure (18 %) konstant gehalten.

Zur Aufarbeitung wird das Pigment abfiltriert, mit 15 l vollentsalztem Wasser gewaschen, bei 110°C 16 Stunden getrocknet und durch ein Sieb mit 32 µm Maschenweite gesiebt.

Man erhält ein karminrotes Pulver mit sehr weichem Hautgefühl, das hervorragend als farbiger Füllstoff für kosmetische Formulierungen, z.B. Presspuder, Lippenstifte sowie Emulsionen und Cremes, geeignet ist.

### Beispiel 6: Mit Titandioxid und Berliner Blau beschichtete Mikrokugeln

250 g sphärische Silica-Alumina-Keramik-Kugeln; Zeeospheres W-210, 1-12 µm; Hersteller 3M) werden in 1,8 l vollentsalztem Wasser suspendiert und unter starkem Rühren auf 75 °C erhitzt. Zu dieser Mischung wird bei pH 2,2 eine Menge von 395 g TiCl₄-Lösung (30 %ig) mit einer Geschwindigkeit von 3,3 ml/min zudosiert und der pH-Wert mit Natronlauge (32 %) konstant gehalten. Nach einer Nachrührzeit von 15 Min. wird der pH-Wert mit Natronlauge auf pH 7,0 eingestellt. Bei diesem pH-Wert wird eine Lösung bestehend aus 2,6 g FeSO₄ x 7 H₂O, 0,06 g Schwefelsäure (ca. 97 %) und 65 g VE-Wasser innerhalb ca. 45 min. zudosiert. Dabei wird der pH-Wert mit Ammoniumhydroxid-Lösung (10 %) konstant gehalten. Anschließend wird innerhalb von ca. 70 min. eine Lösung von 3,64 g K₄[Fe(CN)₆] x 3H₂O in 195 g VE-Wasser zudosiert und 30 min. nachgerührt. Während der gesamten Reaktion wird Luft (4-6 l/h) in die Suspension eingeblasen. Nach beendeter Zugabe der Reaktionslösungen wird 30 Min. nachgerührt.

Das Pigment wird auf einer Filternutsche abgesaugt, mit 30 l VE-Wasser gewaschen, 12h bei 100°C getrocknet und gesiebt (Maschenweite 100 µm).

Man erhält ein blaues Pulver mit sehr weichem Hautgefühl, das hervorragend als farbiger Füllstoff für kosmetische Formulierungen, z.B. Presspuder, Lippenstifte sowie Emulsionen und Cremes, geeignet ist.

### Anwendungsbeispiele

### Beispiel A1: Eye shadow Gel

| **Rohstoff** | | **INCI** | **[%]** |
|---|---|---|---|
| Phase A | | | |
| Timiron Super Gold | (1) | MICA, Cl 77891 (TITANIUM DIOXIDE) | 15,00 |
| Pigment nach Beispiel 1 | | | 7,00 |
| Carbopol Ultrez 21 | (2) | Acrylates/C10-30 Alkyl Acrylate crosspolymer | 0,30 |
| Aloe Vera Powder regular 200x | (3) | Aloe Barbadensis | 0,05 |
| Citronensäure Monohydrat | (1) | Citric Acid | 0,00 |
| Wasser, demineralisiert | | Aqua (Water) | 55,87 |

| Phase B | | | |
|---|---|---|---|
| Triethanolamin 90 % Care | (4) | Triethanolamine, Aqua (Water) | 0,78 |
| Germaben II | (5) | Propylene Glycol, Diazolidinyl Urea, Methylparaben, Propylparaben | 1,00 |
| Glycerin, wasserfrei | (1) | Glycerin | 2,00 |
| Wasser, demineralisiert | | Aqua (Water) | 13,00 |

| Phase C | | | |
|---|---|---|---|
| Lubrajel DV | (6) | Propylene Glycol, Polyglycerylmethacrylate | 5,00 |

### Herstellung:

Aloe Vera Puder im Wasser der Phase A lösen, dann alle Pigmente und die restlichen Inhaltsstoffe bis auf das Carbopol zufügen und dispergieren. Mit einigen Tropfen Citronensäure ansäuern, um die Viskosität zu vermindern, dann Carbopol unter Rühren einstreuen. Nach vollständiger Lösung die vorgelöste Phase B langsam einrühren (nicht homogenisieren) und anschließend Phase C zugeben. Falls erforderlich, den pH-Wert zwischen 7,0-7,5 mit Citronensäurelösung einstellen.

Man erhält eine wasserbasierende Eye Shadow Gel Formulierung mit Aloe Vera. (Schnellsttrocknend und gut mit den Fingern aufzutragen.)

### Bezugsquellen:

(1) Merck KGaA/Rona^{®}
(2) Noveon
(3) Terry Laboratoires, Inc.
(4) BASF AG
(5) ISP Global Technologies
(6) Guardian

### Beispiel A2: Creamy Eyeshadow

| **Rohstoff** | | **INCI** | **[%]** |
|---|---|---|---|
| Phase A | | | |
| Colorona Light Blue | (1) | MICA, Cl 77891 (TITANIUM DIOXIDE) Cl 77510 (FERRIC FERROCYANIDE) | 10,00 |
| Pigment nach Beispiel 1 | | | 15,00 |
| Talkum | (1) | Talc | 12,00 |

| Phase B | | | |
|---|---|---|---|
| Crodamol PMP | (2) | PPG-2Myristyl Ether Propionate | 32,80 |
| Miglyol 812 N | (3) | Caprylic/Capric Triglyceride | 12,00 |
| Syncrowax HGLC | (2) | C18-36 Acid Triglyceride | 10,00 |
| Syncrowax HRC | (2) | Tribehenin | 3,00 |
| Parteck^{®} LUB STA | (1) | Stearic Acid | 3,00 |
| Antaron V-216 | (4) | PVP/Hexadecene Copolymer | 2,00 |
| Oxynex^{®} K flüssig | (1) | PEG-8, Tocopherol, Ascorbyl Palmitate, Ascorbic Acid, Citric Acid | 0,10 |
| Propyl-4-hydroxybenzoat | (1) | Propylparaben | 0,10 |

### Herstellung:

Phase B auf ca. 80 °C erhitzen bis alles geschmolzen ist und unter Rühren auf 65 °C abkühlen. Unter Rühren nun die Inhaltsstoffe der Phase A zugeben und die Masse bei 65 °C in das vorgesehene Packmittel gießen. Auf Raumtemperatur abkühlen lassen.

### Bezugsquellen:

(1) Merck KGaA/Rona^{®}
(2) Croda GmbH
(3) Sasol Germany GmbH
(4) ISP Global Technologies

### Beispiel A3: Gesichtspuder

| **Rohstoff** | | **INCI** | **[%]** |
|---|---|---|---|
| Phase A | | | |
| Pigment nach Beispiel 4 | | | 20,00 |
| Unipure Yellow LC 182 | (1) | Cl 77492 (Iron Oxides) | 1,20 |
| Unipure Red LC 381 | (1) | Cl 77491 (Iron Oxides) | 0,20 |
| Unipure Brown LC 889 | (1) | Cl 77491 (Iron Oxides) Cl 77499 (Iron Oxides) | 0,30 |
| Magnesiumstearat | (2) | Magnesium Stearate | 2,00 |
| Talkum | (2) | Talc | 71,90 |

| Phase B | | | |
|---|---|---|---|
| RonaCare^{®} all-rac-alpha-Tocopherylacetat | (2) | Tocopherylacetate | 0,30 |
| Parfümöl 200 529 | (3) | Parfum | 0,30 |
| Eutanol G | (4) | Octyldodecanol | 3,70 |
| Propyl-4-hydroxybenzoat | (2) | Propylparaben | 0,10 |

### Herstellung:

Die Bestandteile der Phase A in den Mixer (z. B. La Moulinette von Moulinex) geben und 2 x 10 Sekunden mixen. Die Mischung in ein Becherglas überführen, Phase B hinzugeben und mit dem Spatel vorab verrühren. Die Mischung aus Phase A und Phase B wiederum in den Mixer geben und 3 x 10 Sekunden zu einer homogenen Phase verarbeiten.

Der Pressdruck für eine Puderpfanne mit 36 mm Durchmesser beträgt ca. 25 bar.

### Bezugsquellen:

(1) Les Colorants Wackherr
(2) Merck KGaA/Rona^{®}
(3) Fragrance Resources
(4) Cognis GmbH

### Beispiel A4: Mattifying Foundation

| **Rohstoff** | | **INCI** | **[%]** |
|---|---|---|---|
| Phase A | | | |
| Wasser, demineralisiert | | Aqua (Water) | 57,89 |
| Pigment nach Beispiel 3 | | | 6,00 |
| Glycerin (87% reinst) | (1) | Glycerin, Aqua (Water) | 5,00 |
| RonaCare^{®} Ectoin | (1) | Ectoin | 0,30 |
| Keltrol CG-SFT | (2) | Xanthan Gum | 0,15 |
| Triethanolamin 90% Care | (3) | Triethanolamine, Aqua (Water) | 0,13 |

| Phase B | | | |
|---|---|---|---|
| Kronos 1001 | (4) | Cl 77891 (Titanium Dioxide) | 4,92 |
| Unipure Yellow LC 182 | (5) | Cl 77492 (Iron Oxides) | 1,60 |
| Unipure Red LC 381 | (5) | Cl 77491 (Iron Oxides) | 0,20 |
| Unipure Brown LC 889 | (5) | Cl 77491 (Iron Oxides) Cl 77499 (Iron Oxides) | 0,20 |
| Unipure Blue LC 686 | (5) | Cl 77007 (Ultramarin Blue) | 0,08 |

| Phase C | | | |
|---|---|---|---|
| Miglyol 812N | (6) | Caprylic/Capric Triglyceride | 7,00 |
| Eutanol G | (7) | Octyldodecanol | 4,00 |
| Montanov 202 | (8) | Arachidyl Alcohol, Behenyl Alcohol, Arachidylglucoside | 4,00 |
| Avocadoöl | (9) | Persea Gratissima (Avocado Oil) | 2,00 |
| Eusolex^{®} 9020 | (1) | Butyl Methoxydibenzoylmethane | 1, 50 |
| Hydrolite-5 | (10) | Pentylene Glycol | 1,20 |
| Bentone Gle GTCC V | (11) | Stearalkonium Hectorite, Propylene Carbonate, Caprylic/Capric Triglyceride | 1,00 |
| RonaCare^{®} all-rac-alpha-Tocopherylacetat | (2) | Tocopherylacetate | 0,50 |
| Phenonip | (12) | Phenoxyethanol, Butylparaben, Ethylparaben, Propylparaben, Methylparaben | 0,40 |
| Oxynex^{®} K flüssig | (1) | PEG-8, Tocopherol, Ascorbyl Palmitate, Ascorbic Acid, Citric Acid | 0,03 |

| Phase D | | | |
|---|---|---|---|
| Simulgel EG | (8) | Sodium Acrylate/Sodium Acryloyldimethyltaurate Copolymer, Isohexadecane, Polysorbate 80 | 0,60 |

| Phase E | | | |
|---|---|---|---|
| Wasser, demineralisiert | | Aqua (Water) | 1,00 |

### Herstellung:

Keltrol langsam ins Wasser der Phase A geben und dispergieren. Restliche Bestandteile der Phase A unter Rühren einstreuen. Die Bestandteile der Phase B zur Phase A geben und mit dem Ultra-Turrax T25 (Stufe rot-blau, 13500-20500 Upm) 3 min homogenisieren und auf Agglomerate prüfen. Phase A/B und Phase C separat auf 75 °C erhitzen. Unter Rühren Phase C zu Phase A/B geben und 2 min mit dem Ultra-Turrax T25 (Stufe gelb-grün, 8000-9500 Upm) homogenisieren. Zwischen 55-60 °C Phase D zugeben, bei 40 °C Phase E zugeben, unter weiterem Rühren auf Raumtemperatur abkühlen; pH-Wert auf 7,0 mit 30 %iger Citronensäure einstellen. Dann in geeignete Behältnisse abfüllen.

Man erhält eine leichte, schwach deckende Foundation, die für alle Hauttypen geeignet ist. Avocadoöl, Vitamin-E-Acetat und das zellschützende RonaCare^{®} Ectoin unterstützen die hautpflegende Wirkung.

### Bezugsquellen:

(1) Merck KGaA/Rona^{®}
(2) C.P. Kelco
(3) BASF AG
(4) Kronos International Inc.
(5) Les Colorants Wackherr
(6) Sasol Germany GmbH
(7) Cognis GmbH
(8) Seppic
(9) Gustav Heess GmbH
(10) Symrise
(11) Elementis Specialities
(12) Clariant GmbH

### Beispiel A5: Body Lotion

| **Rohstoff** | | **INCI** | **[%]** |
|---|---|---|---|
| Phase A | | | |
| Aloe Vera Gel 10x declorized | (1) | ALOE BARBADENSIS | 2,00 |
| D-Panthenol | (2) | PANTHENOL | 0,40 |
| Pigment aus Beispiel 1 | | | 6,00 |
| RonaCare^{®} Allantoin | (3) | ALLANTOIN | 0,20 |
| Glycerin, wasserfrei | (3) | GLYCERIN | 4,00 |
| Wasser, demineralisiert | | AQUA (WATER) | 67,57 |

| Phase B | | | |
|---|---|---|---|
| Protelan AGL 95/C | (4) | SODIUM COCOYL GLUTAMATE | 6,00 |
| Cosmacol EMI | (5) | DI-C12-13 ALKYL MALATE | 3,00 |
| Eutanol G | (6) | Octyldodecanol | 3,00 |
| Jojobaöl | (7) | SIMMONDSIA CHINENSIS (JOJOBA OIL) | 1,50 |
| Tegosoft TN | (8) | C12-15 Alkyl Alkyl benzoate | 1,50 |
| Carbopol ETD 2020 | (9) | Acrylates/C10-30 Alkyl Acrylate Crosspolymer | 0,60 |
| Phenonip | (10) | Phenoxyethanol, Butylparaben, Ethylparaben, Propylparaben, Methylparaben | 0,60 |
| RonaCare^{®} Bisabolol | (3) | Bisabolol | 0,50 |
| RonaCare^{®} all-rac-alpha-Tocopherylacetat | (3) | Tocopherylacetate | 0,50 |
| Oxynex^{®} ST Liquid | (3) | Diethylhexyl Syringylidenemalonate, Caprylic/Capric Triglyceride | 0,50 |
| Cremophor RH 410 | (11) | PEG-40 Hydrogenated Castor Oil | 0,30 |
| Oxynex^{®} K flüssig | | PEG-8, Tocopherol, Ascorbyl Palmitate, Ascorbic Acid, Citric Acid | 0,03 |

| Phase C | | | |
|---|---|---|---|
| Parfümöl Lifetime DH10255/1 | (12) | Parfum | 0,50 |

| Phase D | | | |
|---|---|---|---|
| Wasser, demineralisiert | | Aqua (Water) | 1,00 |
| Germal 115 | (13) | Imidazolidinyl Urea | 0,30 |

### Herstellung:

Aloe Vera und RonaCare^{®} Allantoin im Wasser der Phase A unter Rühren vorlösen, dann die anderen Bestandteile der Phase A zugeben und auf 60 °C erhitzen, Jojobaöl, Oxynex K flüssig, Cosmacol EMI, Eutanol G und Tegosoft TN in einem Rührgefäß vorlegen, dann Carbopol mit der Dispergierscheibe (ca. 700 U/min, 20 min) dazu homogen einarbeiten. Dann die restlichen Bestandteile der Phase B zufügen und alles zu einer homogenen Mischung verrühren, wobei Protelan AGL 95/C erst ganz zum Schluss zur Phase B zugegeben wird, um einen übermäßigen Lufteintrag zu vermeiden. Phase A bei 60 °C mit Hilfe der Dispergierscheibe langsam in Phase B (RT) einemulgieren. Phase C und D zufügen, danach 4 min mit dem Ultra-Turrax T50, Stufe 4 homogenisieren. Auf Raumtemperatur abkühlen.
pH (23 °C) = 5,5 - 6,0
Viskosität: Brookfield DV II + Helipath, Spindel C, 5 Rpm, 24 °C = 11200 mPa s

### Bezugsquellen:

(1) Terry Laboratoires
(2) Alfa Aesar GmbH & Co. KG
(3) Merck KGaA/Rona^{®}
(4) Zschimmer & Schwarz GmbH & Co.
(5) Nordmann, Rassmann GmbH & Co.
(6) Cognis GmbH
(7) Gustav Heess GmbH
(8) Evonik Goldschmidt GmbH
(9) Noveon
(10) Clariant GmbH
(11) BASF AG
(12) Parfex
(13) ISP Global Technologies

## Patentansprüche

1. Pigmente basierend auf sphärischen Partikeln, **dadurch gekennzeichnet, dass** die sphärischen Partikel ausgewählt sind aus der Gruppe Magnesiumsilikat, Aluminiumsilikat, Alkalialuminiumsilikate, Erdalkalialuminiumsilikate und deren Kombinationen und einen Partikeldurchmesser von 0,1 - 100 µm aufweisen und auf der Oberfläche (1. Schicht) mit Agglomeraten ausgewählt aus der Gruppe ZrO₂, ZnO, Al₂O₃, TiO₂, Fe₂O₃, Fe₃O₄, FeOOH oder mit BaSO₄ oder Gemische davon beschichtet sind, und optional mit einer weiteren Schicht (2. Schicht) aus einem Metalloxid oder Metalloxidgemisch, einem Farblack oder Berliner Blau belegt sind.

2. Pigmente nach Anspruch 1, **dadurch gekennzeichnet, dass** die sphärischen Partikel Magnesium- oder Alkalialuminiumsilikate sind.

3. Pigmente nach Anspruch 1 oder 2, **dadurch gekennzeichnet, dass** die sphärischen Partikel Substratgemische sind ausgewählt aus
Alkalialuminiumsilikat und Magnesiumsilikat
Alkalialuminiumsilikat und Siliziumdioxid oder
Alkalialuminiumsilikat und Erdalkalialuminiumsilikat.

4. Pigmente nach einem oder mehreren der Ansprüche 1 bis 3, **dadurch gekennzeichnet, dass** die sphärischen Partikel eine äußere Schicht (Deckschicht) aus SiO₂ aufweisen.

5. Pigmente nach einem oder mehreren der Ansprüche 1 bis 4, **dadurch gekennzeichnet, dass** die 1. Schicht eine mittlere Schichtdicke von 0,01-2 µm aufweist.

6. Pigmente nach einem oder mehreren der Ansprüche 1 bis 5, **dadurch gekennzeichnet, dass** die äußere Schicht (Deckschicht) eine mittlere Dicke von 0,01 - 1 µm aufweist.

7. Pigmente nach einem oder mehreren der Ansprüche 1 bis 6, **dadurch gekennzeichnet, dass** der Gewichtsanteil des Farblackes am Gesamtpigment 0,1-30 % beträgt.

8. Pigmente nach einem oder mehreren der Ansprüche 1 bis 7, **dadurch gekennzeichnet, dass** die sphärischen Partikel (Basissubstrat) folgende Belegung auf der Oberfläche aufweisen:
sphärisches Basissubstrat + TiO₂
sphärisches Basissubstrat + TiO₂ + SiO₂
sphärisches Basissubstrat + TiO₂/Fe₂O₃
sphärisches Basissubstrat + TiO₂/Fe₂O₃ + SiO₂
sphärisches Basissubstrat + Fe₂O₃ + SiO₂
sphärisches Basissubstrat + FeO(OH) + SiO₂
sphärisches Basissubstrat + Fe₃O₄ + SiO₂
sphärisches Basissubstrat + TiFe₂O₅ + SiO₂
sphärisches Basissubstrat + TiO₂ + Fe₂O₃ + SiO₂
sphärisches Basissubstrat + TiO₂ + Fe₃O₄ + SiO₂
sphärisches Basissubstrat + TiO₂ + Fe₄[Fe(CN)₆]₃
sphärisches Basissubstrat + TiO₂ + Fe₄[Fe(CN)ₑ]₃ + SiO₂
sphärisches Basissubstrat + TiO₂ + Carmin-Lack
sphärisches Basissubstrat + TiO₂ + Carmin-Lack + SiO₂.

9. Pigmente nach einem oder mehreren der Ansprüche 1 bis 8, **dadurch gekennzeichnet, dass** die Pigmente eine Ölzahl (bestimmt nach DIN ISO 787/5-1980 (E)) von 10 - 200 aufweisen.

10. Pigmente nach einem oder mehreren der Ansprüche 1 bis 9, **dadurch gekennzeichnet, dass** die Pigmente eine BET-Oberfläche (bestimmt nach DIN 9277:2003-05), von 1 - 200 m²/g aufweisen.

11. Pigmente nach einem oder mehreren der Ansprüche 1 bis 10, **dadurch gekennzeichnet, dass** sie zur Erhöhung der Licht-, Temperatur- und Wetterstabilität zusätzlich eine äußere Schutzschicht aufweisen.

12. Verfahren zur Herstellung der Pigmente nach einem oder mehreren der Ansprüche 1 bis 11, **dadurch gekennzeichnet, dass** die sphärischen Partikel durch Ausfällung des Schichtmaterials in wässriger Suspension durch weitgehend gleichmäßige und gleichzeitige oder zeitversetzte Zugabe der Lösungen der Agglomerat- bzw. der Schicht-bildenden Rohstoffe in Gegenwart von Säuren bzw. Basen mit den Agglomeraten bzw. Schichtmaterialien belegt, abfiltriert, gewaschen, getrocknet, gegebenenfalls kalziniert und gesiebt werden.

13. Verwendung der Pigmente nach einem oder mehreren der Ansprüche 1 bis 11 in Farben, Lacken, Druckfarben, Sicherheitsdruckfarben, Kunststoffen, keramischen Materialien, Gläsern, in kosmetischen Formulierungen, als Tracer, als Füllstoff und zur Herstellung von Pigmentpräparationen und Trockenpräparaten.

14. Verwendung der Pigmente nach Anspruch 13 in Nagellacken, Lippenstiften, Presspudern, Gelen, Lotionen, Seifen, Zahnpasta, in Industrielacken und Pulverlacken, Kunststoffen und in der Keramik.

15. Verwendung der Pigmente nach Anspruch 13 für Personal Care Applications.

16. Formulierungen enthaltend ein Pigment nach einem oder mehreren der Ansprüche 1 bis 11.

17. Formulierungen nach Anspruch 16, **dadurch gekennzeichnet, dass** sie neben dem Pigment nach einem oder mehreren der Ansprüche 1 bis 11 mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsions-stabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

## Claims

1. Pigments based on spherical particles, **characterised in that** the spherical particles are selected from the group magnesium silicate, aluminium silicate, alkali-metal aluminium silicates, alkaline-earth metal aluminium silicates and combinations thereof and have a particle diameter of 0.1 - 100 µm and are coated on the surface (1st layer) with agglomerates selected from the group ZrO₂, ZnO, Al₂O₃, TiO₂, Fe₂O₃, Fe₃O₄, FeOOH or with BaSO₄ or mixtures thereof, and are optionally coated with a further layer (2nd layer) comprising a metal oxide or metal-oxide mixture, a lake or Prussian Blue.

2. Pigments according to Claim 1, **characterised in that** the spherical particles are magnesium or alkali-metal aluminium silicates.

3. Pigments according to Claim 1 or 2, **characterised in that** the spherical particles are substrate mixtures selected from
alkali-metal aluminium silicate and magnesium silicate
alkali-metal aluminium silicate and silicon dioxide or
alkali-metal aluminium silicate and alkaline-earth metal aluminium silicate.

4. Pigments according to one or more of Claims 1 to 3, **characterised in that** the spherical particles have an outer layer (top layer) comprising SiO₂.

5. Pigments according to one or more of Claims 1 to 4, **characterised in that** the 1st layer has an average layer thickness of 0.01-2 µm.

6. Pigments according to one or more of Claims 1 to 5, **characterised in that** the outer layer (top layer) has an average thickness of 0.01 - 1 µm.

7. Pigments according to one or more of Claims 1 to 6, **characterised in that** the proportion by weight of the lake in the entire pigment is 0.1-30%.

8. Pigments according to one or more of Claims 1 to 7, **characterised in that** the spherical particles (base substrate) have the following coating on the surface:
spherical base substrate + TiO₂
spherical base substrate + TiO₂ + SiO₂
spherical base substrate + TiO₂/Fe₂O₃
spherical base substrate + TiO₂/Fe₂O₃ + SiO₂
spherical base substrate + Fe₂O₃ + SiO₂
spherical base substrate + FeO(OH) + SiO₂
spherical base substrate + Fe₃O₄ + SiO₂
spherical base substrate + TiFe₂O₅ + SiO₂
spherical base substrate + TiO₂ + Fe₂O₃ + SiO₂
spherical base substrate + TiO₂ + Fe₃O₄ + SiO₂
spherical base substrate + TiO₂ + Fe₄[Fe(CN)₆]₃
spherical base substrate + TiO₂ + Fe₄[Fe(CN)₆]₃ + SiO₂
spherical base substrate + TiO₂ + carmine lake
spherical base substrate + TiO₂ + carmine lake + SiO₂.

9. Pigments according to one or more of Claims 1 to 8, **characterised in that** the pigments have an oil absorption value (determined in accordance with DIN ISO 787/5-1980 (E)) of 10 - 200.

10. Pigments according to one or more of Claims 1 to 9, **characterised in that** the pigments have a BET surface area (determined in accordance with DIN 9277:2003-05) of 1 - 200 m²/g.

11. Pigments according to one or more of Claims 1 to 10, **characterised in that** they additionally have an outer protective layer in order to increase the light, temperature and weather stability.

12. Process for the preparation of the pigments according to one or more of Claims 1 to 11, **characterised in that** the spherical particles are coated with the agglomerates or layer materials by precipitation of the layer material in aqueous suspension by substantially uniform and simultaneous or non-simultaneous addition of the solutions of the agglomerate- or layer-forming raw materials in the presence of acids or bases, filtered off, washed, dried, optionally calcined and sieved.

13. Use of the pigments according to one or more of Claims 1 to 11 in paints, coatings, printing inks, security printing inks, plastics, ceramic materials, glasses, in cosmetic formulations, as tracer, as filler and for the preparation of pigment preparations and dry preparations.

14. Use of the pigments according to Claim 13 in nail varnishes, lipsticks, compact powders, gels, lotions, soaps, toothpastes, in industrial coatings and powder coatings, plastics and in ceramics.

15. Use of the pigments according to Claim 13 for personal care applications.

16. Formulations comprising a pigment according to one or more of Claims 1 to 11.

17. Formulations according to Claim 16, **characterised in that**, besides the pigment according to one or more of Claims 1 to 11, they comprise at least one constituent selected from the group of the absorbents, astringents, antimicrobial substances, antioxidants, antiperspirants, antifoaming agents, antidandruff active compounds, antistatics, binders, biological additives, bleaches, chelating agents, deodorisers, emollients, emulsifiers, emulsion stabilisers, dyes, humectants, film-formers, fillers, fragrances, flavours, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, viscosity regulators, perfume and vitamins.

## Revendications

1. Pigments à base de particules sphériques, **caractérisés en ce que** les particules sphériques sont choisies dans le groupe constitué par le silicate de magnésium, le silicate d'aluminium, les silicates de métaux alcalins et d'aluminium, les silicates de métaux alcalino-terreux et d'aluminium et des combinaisons de ceux-ci, et possèdent un diamètre de particules de 0,1 - 100 µm et sont revêtus à la surface (1 ère couche) par des agglomérats choisis dans le groupe constitué par ZrO₂, ZnO, Al₂O₃, TiO₂, Fe₂O₃, Fe₃O₄, FeOOH ou par BaSO₄ ou des mélanges de ceux-ci, et sont éventuellement revêtus d'une autre couche (2ème couche) comprenant un oxyde de métal ou un mélange métal-oxyde, une laque ou du Bleu de Prusse.

2. Pigments selon la revendication 1, **caractérisés en ce que** les particules sphériques sont des silicates de magnésium ou de métaux alcalins et d'aluminium.

3. Pigments selon la revendication 1 ou 2, **caractérisés en ce que** les particules sphériques sont des mélanges de substrats choisis parmi le silicate de métaux alcalins et d'aluminium et le silicate de magnésium
le silicate de métaux alcalins et d'aluminium et le dioxyde de silicium ou
le silicate de métaux alcalins et d'aluminium et le silicate de métaux alcalino-terreux et d'aluminium.

4. Pigments selon l'une ou plusieurs parmi les revendications 1 à 3, **caractérisés en ce que** les particules sphériques possèdent une couche externe (couche supérieure) comprenant du SiO₂.

5. Pigments selon l'une ou plusieurs parmi les revendications 1 à 4, **caractérisés en ce que** la 1 ère couche possède une épaisseur moyenne de couche de 0,01-2 µm.

6. Pigments selon l'une ou plusieurs parmi les revendications 1 à 5, **caractérisés en ce que** la couche externe (couche supérieure) possède une épaisseur moyenne de 0,01 - 1 µm.

7. Pigments selon l'une ou plusieurs parmi les revendications 1 à 6, **caractérisés en ce que** la proportion en poids de la laque dans l'ensemble du pigment est de 0,1-30%.

8. Pigments selon l'une ou plusieurs parmi les revendications 1 à 7, **caractérisés en ce que** les particules sphériques (substrat de base) possèdent le revêtement suivant à la surface :
substrat de base sphérique + TiO₂
substrat de base sphérique + TiO₂ + SiO₂
substrat de base sphérique + TiO₂/Fe_{2O3}
substrat de base sphérique + TiO₂/Fe₂O₃ + SiO₂
substrat de base sphérique + Fe₂O₃ + SiO₂
substrat de base sphérique + FeO(OH) + SiO₂
substrat de base sphérique + Fe₃O₄ + SiO₂
substrat de base sphérique + TiFe₂O₅ + SiO₂
substrat de base sphérique + TiO₂ + Fe₂O₃ + SiO₂
substrat de base sphérique + TiO₂ + Fe₃O₄ + SiO₂
substrat de base sphérique + TiO₂ + Fe₄[Fe(CN)₆]₃
substrat de base sphérique + TiO₂ + Fe₄[Fe(CN)₆]₃ + SiO₂
substrat de base sphérique + TiO₂ + laque carminée
substrat de base sphérique + TiO₂ + laque carminée + SiO₂.

9. Pigments selon l'une ou plusieurs parmi les revendications 1 à 8, **caractérisés en ce que** les pigments possèdent une valeur d'absorption d'huile (déterminée selon DIN ISO 787/5-1980 (E)) de 10 - 200.

10. Pigments selon l'une ou plusieurs parmi les revendications 1 à 9, **caractérisés en ce que** les pigments possèdent une surface BET (déterminée selon DIN 9277:2003-05) de 1 - 200 m²/g.

11. Pigments selon l'une ou plusieurs parmi les revendications 1 à 10, **caractérisés en ce qu'**ils possèdent en outre une couche protectrice externe afin d'augmenter la stabilité vis-à-vis de la lumière, de la température et des intempéries.

12. Procédé de préparation des pigments selon l'une ou plusieurs parmi les revendications 1 à 11, **caractérisé en ce que** les particules sphériques sont revêtues par les agglomérats ou les matériaux de couche par précipitation du matériau de couche en suspension aqueuse par addition sensiblement uniforme et simultanée ou non simultanée des solutions des matières premières d'agglomérats ou formatrices de couches en présence d'acides ou de bases, filtrées, lavées, séchées éventuellement calcinées et tamisées.

13. Utilisation des pigments selon l'une ou plusieurs parmi les revendications 1 à 11, dans des peintures, des revêtements, des encres d'impression, des encres d'impression de sécurité, des matières plastiques, des matériaux de céramique, des verres, dans des formulations cosmétiques, comme traceur, comme charge et pour la préparation de préparations de pigments et de préparations sèches.

14. Utilisation des pigments selon la revendication 13, dans des vernis à ongles, des rouges à lèvres, des poudres compactes, des gels, des lotions, des savons, des dentifrices, dans des revêtements industriels et des revêtements en poudre, des matières plastiques et dans des céramiques.

15. Utilisation des pigments selon la revendication 13, pour des applications de soin personnel.

16. Formulations comprenant un pigment selon l'une ou plusieurs parmi les revendications 1 à 11.

17. Formulations selon la revendication 16, **caractérisées en ce que**, outre le pigment selon l'une ou plusieurs parmi les revendications 1 à 11, elles comprennent au moins un constituant choisi dans le groupe constitué par les absorbants, les astringents, les substances antimicrobiennes, les antioxydants, les anti-transpirants, les agents antimousse, les composés actifs antipelliculaires, les agents anti-électrostatiques, les liants, les additifs biologiques, les agents de blanchiment, les agent chélateurs, les désodorisants, les émollients, les émulsifiants, les stabilisants d'émulsion, les colorants, les humectants, les agents filmogènes, les charges, les fragrances, les arômes, les insectifuges, les conservateurs, les agents anticorrosion, les huiles cosmétiques, les solvants, les oxydants, les constituants végétaux, les substances tampon, les agents réducteurs, les agents tensioactifs, les gaz propulseurs, les opacifiants, les filtres anti-UV et agents anti-UV, les agents dénaturants, les régulateurs de viscosité, les parfums et les vitamines.
